# EUROPEAN PATENT APPLICATION

(11) **EP 4 679 319 A1**
(43) Date of publication of application: **14.01.2026**
(21) Application number: 24187429.6
(22) Date of filing: 09.07.2024
(51) Int. Cl.: G06K 13/00, A61B 90/90, A61B 90/98, G06K 17/00, G06Q 10/0833, G06Q 50/04, G16H 40/63

(54) **SYSTEM AND METHOD FOR LOCALIZATION OF TAGGED MEDICAL INJECTION DEVICES IN A NEST**

(71) Applicant: Becton, Dickinson and Company, Franklin Lakes, NJ 07417-1880 (US)
(72) Inventor: GARREC, Ronan, 38640 CLAIX (FR); LUXEMBOURG, David, 38000 Grenoble (FR); MESSAOUD, Mourad, 38000 Grenoble (FR)
(74) Representative: Germain Maureau

(57) **Abstract**

Provided herein is a system for localizing tagged medical injection devices in a nest. The system includes a conveyor and associated holders that convey and retain injection devices along a conveying path. A device reads each tagged medical injection device to retrieve a unique device identifier (UDI) from the tag. A transfer device transfers each injection device from the conveyor system to a nest having a nest UDI associated therewith. A processor in the system receives a UDI for each injection device and, upon the injection device being transferred to the nest, associates the UDI with a location in the nest at which the injection device is stored and with the nest UDI, so as to register the location of each tagged medical injection device within the nest.

## Description

### BACKGROUND OF THE INVENTION

### Field of the Invention

The present disclosure relates generally to tagged medical injection devices retained in a nest and, more particularly, to a system and method for localizing tagged medical injection devices or components thereof within the nest, as the devices are transferred into the nest during production.

### Description of Related Art

Medical injection devices, such as syringes, are used in a variety of environments for administering fluids, such as medications or other drugs, to a patient. Syringes may be provided as prefilled or pre-fillable syringes, which provide the convenience of rapidly delivering the liquid therein to a patient without the need to first aspirate the medication from another container and meter its volume. A syringe will typically include a syringe barrel having a distal end and a proximal end. A needle is connected at the distal end for injecting fluid into the patient and a plunger assembly is inserted through the proximal end that is movable within the barrel to force fluid out from the syringe through the needle.

Components of the syringes as described above often need to be transported from one site to another site during or after manufacturing. For instance, syringe barrels (which, as used herein, may be understood to include the barrel, the needle, and a needle cover) may be transported from a manufacturing site to a site at which the barrels may be filled with a "medicinal fluid," which can refer to a medication or another therapeutic agent used for treatment of chronic or acute conditions, as are known in the art. The filling of the syringe barrels is an automated process that is performed by a fill and finish machine, which dispenses fluid into the syringe barrels in an automated fashion.

During transportation and filling, the syringe barrels may be supported by a holding device, such as a tray or "nest." As known in the art, a nest can be a plate-shaped tray configured to support multiple medical containers in an upright orientation. Nests can comprise multiple "chimneys" aligned according to predetermined rows or a predetermined pattern, with each chimney defining a receptacle or opening configured to receive one medical container. When inserted into and through the receptacle, the medical container is held in the upright orientation in a direction substantially orthogonal to the nest. Once retained in a nest, the syringes may be transferred to an automated filling station, where the syringes are filled with a medicinal fluid by the fill and finish machine.

In an effort to automate the manufacturing of syringes (including filling of the syringes with medicinal fluid) and provide for further traceability of the syringes from the manufacturing process until the final labeling, the final use, or the disposal of the medical devices, it is becoming more common for such syringes to implement an identifying element or tag thereon that provides for identification and tracing of the syringes. As non-limiting examples, a syringe may include a radio frequency identification (RFID) tag or two-dimensional code (e.g., data-matrix or QR code) tag thereon having a unique device identifier (UDI) for the syringe - generally, "tagged" syringes. As relates to the manufacturing process, identifying elements may be provided in/on the syringes (e.g., integrated into or applied onto the needle shield thereof) at the site of initial manufacturing, with the tagged syringes (i.e., tagged syringe barrels) then loaded into a nest (and tub) for subsequent shipping and filling.

As known in the art, it is desirable to associate a plurality of tagged syringes with a particular nest in which they are stored. This association may be provided by identifying an RFID tag on the nest and each syringe stored in that nest. For a given nest, the association (so called "aggregation") may be provided as a table with the UDI of the nest and a list of the UDIs of all syringes in that nest - with this table being stored in a database. This aggregation of the UDIs of the syringes with the UDI of the nest is advantageous, as it may simplify subsequent processing/filling of the syringes at a customer location, as the customer may only need to read the tagged nest to determine the UDI of each syringe barrel stored in the nest (rather than reading the UDI of each syringe). The customer may thus be able to efficiently control the fill and finish machine when filling the tagged syringes.

In some methods/techniques for performing data aggregation for syringes and nests, specifically where the syringes and nest include RFID tags thereon, it is known to make a "mass reading" of the syringes stored in the nest. This mass reading can be performed using a far field RFID antenna and a RFID reader, with all the RFID-tagged syringes in a nest being read collectively, in one reading, and associated with the RFID-tagged nest.

While the mass reading technique is typically efficient in aggregating syringe and nest UDIs, it is recognized that there are several drawbacks in using such a mass reading technique. As one example, use of a mass reading technique to associate the UDIs of the syringes with the UDI of the nest does not allow for a determination/identification of the location of each tagged syringe barrel within the nest (i.e., the specific chimney in which it is retained), which may be desirable for the customer when filling the RFID-tagged syringes via an automated fill and finish machine, in order to dispense an appropriate type and quantity of medicinal fluid into each syringe barrel retained within a nest. As another example, use of a mass reading technique to associate the UDIs of the syringes with the UDI of the nest is not feasible when the identifying element on each syringe is a two-dimensional code identifier (e.g., data matrix-type or QR code), as such identifiers are read by a camera and not all of the two-dimensional codes of the syringes may be visible/readable by the camera with the syringes positioned/retained in the nest.

Accordingly, a need exists in the art for a system and method of aggregating tagged syringes with a tagged nest, including for both RFID-tagged syringes/nests and QR-tagged syringes/nests. The system and method would desirably be capable of localizing each tagged syringe barrel within the nest, with such localization of the syringes aiding in an automated fill and finish manufacturing process.

### SUMMARY OF THE INVENTION

Provided herein is a system for localizing tagged medical injection devices in a nest. The system includes a conveyor system configured to convey a plurality of tagged medical injection devices along a conveying path, the conveyor system comprising a plurality of holders, with each of the plurality of holders identified by a holder number and configured to receive a respective tagged medical injection device of the plurality of tagged medical injection devices. The system also includes a device reader positioned at a reading location along the conveying path, the device reader configured to perform a reading of a tag of each of the plurality of tagged medical injection devices upon each respective tagged medical injection device passing the reading location, wherein reading the tag comprises reading a unique device identifier (UDI) from the tag. The system further includes a transfer device positioned adjacent the conveyor system, at a location downstream from the reading location, the transfer device configured to individually transfer each of the plurality of tagged medical injection devices from the conveyor system to a nest, the nest including a nest tag having a nest UDI associated therewith. The system still further includes at least one processor coupled to a memory and configured to receive the UDI for a respective tagged medical injection device from the device reader and, upon the tagged medical injection device being transferred to the nest, associating the UDI of the tagged medical injection device with a location in the nest at which the tagged medical injection device is stored and with the nest UDI, so as to register the location of each of the plurality of tagged medical injection devices within the nest.

In accordance with some aspects of the disclosure, the at least one processor is configured to associate the UDI for a respective tagged medical injection device with the holder number of the holder carrying the tagged medical injection device.

In accordance with some aspects of the disclosure, the at least one processor is configured to receive a fail signal from the device reader if the device reader is unable to read the tag of a respective tagged medical injection device as it passes the reading location and associate the fail signal with the holder number of the holder that passed the reading location with no tag reading occurring.

In accordance with some aspects of the disclosure, the at least one processor is operably connected to the transfer device and is further configured to cause the transfer device to transfer a respective tagged medical injection device from a respective holder to the nest, when the holder number for the holder is determined to have a UDI for a tagged medical injection device associated therewith, or cause the transfer device to discard a respective tagged medical injection device from a respective holder, when the holder number for the holder is determined to have a fail signal associated therewith.

In accordance with some aspects of the disclosure, the nest comprises a support plate and a plurality of chimneys extending outwardly from the support plate, each of the plurality of chimneys configured to retain a tagged medical injection device therein, the plurality of chimneys arranged in a plurality of columns, with each of the plurality of columns comprising a plurality of seats.

In accordance with some aspects of the disclosure, associating the location in the nest at which the tagged medical injection device is stored with the UDI for the tagged medical injection device comprises registering the column and seat at which the tagged medical injection device is stored with the UDI.

In accordance with some aspects of the disclosure, the number of chimneys in the nest is greater than the number of holders in the conveyor system, and wherein the conveying path comprises a loop, and wherein each of the holders passes by the reading location once per time traversing about the loop, with a respective tagged medical injection device being loaded into a respective holder at a beginning of the loop.

In accordance with some aspects of the disclosure, the processor is configured to generate a shift register comprising a plurality of data entries, wherein each data entry comprises a loop number, the holder number, the medical injection device UDI, the nest UDI, and the location in the nest for the medical injection device associated with the medical injection device UDI.

In accordance with some aspects of the disclosure, the system further comprises at least one trigger sensor configured to detect when a respective tagged medical injection device is at the reading location, wherein for each tagged medical injection device on the conveyor system, the at least one sensor is configured to transmit a trigger signal to the device reader to cause the device reader to read the tag of the tagged medical injection device in response to detecting that the medical injection device is at the reading location, and wherein the device reader is configured to read the tag on the respective tagged medical injection device in response to receiving the trigger signal from the at least one sensor.

In accordance with some aspects of the disclosure, the tag comprises a RFID tag, and the device reader comprises an antenna configured to receive a signal response from the RFID tag of a respective medical injection device and a RFID reader operably connected to the antenna, to receive the signal response and read the UDI therefrom.

In accordance with some aspects of the disclosure, the tag comprises a two-dimensional code tag, and the device reader comprises an imaging system including a camera configured to acquire an image of the two-dimensional code tag of a respective medical injection device and an image processor configured to receive the image and determine the UDI from the image.

In accordance with some aspects of the disclosure, the system further comprises a loading device positioned adjacent the conveyor system, at a location upstream from the reading location, the loading device configured to load a respective tagged medical injection device into a respective holder.

In accordance with some aspects of the disclosure, the system further comprises a nest reader configured to read the nest tag to obtain the nest UDI, and wherein the processor receives the nest UDI from the nest reader.

In accordance with some aspects of the disclosure, the plurality of tagged medical injection devices comprise a plurality of syringe barrels.

Also provided herein is a method for localizing tagged medical injection devices in a nest. The method includes conveying each of a plurality of tagged medical injection devices, via a conveyor system including a plurality of holders, along a conveying path, each of the plurality of tagged medical injection devices comprising a tag thereon having a device unique identifier (UDI). The method also includes performing a reading of each of the plurality of tagged medical injection devices at a reading location along the conveying path, via a device reader, as each respective tagged medical injection device passes the reading location, and associating, via at least one processor, the UDI of each respective tagged medical injection device with a holder number of a respective holder retaining the tagged medical injection device. The method further includes transferring the each of the plurality of tagged medical injection devices from the conveyor system to a nest, via a transfer device positioned adjacent the conveyor system and at a location downstream from the reading location, the nest including a nest tag having a nest UDI associated therewith. The method still further includes, as each of the plurality of tagged medical injection devices is being transferred to the nest, associating, via the at least one processor, the UDI of a respective tagged medical injection device with a location in the nest at which the tagged medical injection device is stored and with the nest UDI, so as to register the location of each of the plurality of tagged medical injection devices within the nest.

In accordance with some aspects of the disclosure, the nest comprises a plurality of chimneys each configured to retain a tagged medical injection device therein, the plurality of chimneys arranged in a plurality of columns, with each of the plurality of columns comprising a plurality of seats, and wherein registering the location of each of the plurality of tagged medical injection devices comprises registering the column and seat of each of the plurality of tagged medical injection devices.

In accordance with some aspects of the disclosure, performing a reading of each of the plurality of tagged medical injection devices comprises one of reading an RFID tag of each of the plurality of tagged medical injection devices via an RFID reader, or reading a two-dimensional code on each of the plurality of tagged medical injection devices via an imaging system.

In accordance with some aspects of the disclosure, the method further includes generating a fail signal from the device reader if the device reader is unable to read the tag of a respective tagged medical injection device as it passes the reading location and associating, via the at least one processor, the fail signal with the holder number of the holder that passed the reading location with no tag reading occurring.

In accordance with some aspects of the disclosure, the method further includes causing, via the at least one processor, the transfer device to transfer a respective tagged medical injection device from a respective holder to the nest, when the holder number for the holder is determined to have a UDI for a tagged medical injection device associated therewith, or causing, via the at least one processor, the transfer device to discard a respective tagged medical injection device from a respective holder, when the holder number for the holder is determined to have a fail signal associated therewith.

In accordance with some aspects of the disclosure, in associating the UDI of a respective tagged medical injection device with the location in the nest at which the tagged medical injection device is stored and with the nest UDI, the at least one processor generates a shift register comprising a plurality of data entries, wherein each data entry comprises the holder number, the medical injection device UDI, the nest UDI, and the location in the nest for the medical injection device associated with the medical injection device UDI.

In accordance with some aspects of the disclosure, conveying each of the plurality of tagged medical injection devices along the conveying path comprises conveying each of the plurality of tagged medical injection devices along a loop, wherein each of the holders passes by the reading location once per time traversing about the loop, with a respective tagged medical injection device being loaded into a respective holder at a beginning of the loop, and wherein each data entry in the indexing shift register further comprises a loop number.

In accordance with some aspects of the disclosure, the method further includes detecting when a respective tagged medical injection device is at the reading location using at least one trigger sensor and, in response to detecting that the tagged medical injection device is at the reading location, transmitting a trigger signal from the at least one trigger sensor to the device reader to cause the device reader to read the tag of a respective tagged medical injection device.

### BRIEF DESCRIPTION OF THE DRAWINGS

FIG. 1A is a perspective view of a RFID-tagged syringe, with which embodiments of the disclosure may be implemented;
FIG. 1B is an exploded view of the syringe of FIG. 1A;
FIG. 2 is a perspective view of a nest for storing and shipping a plurality of medical components, such as the syringe of FIGS. 1A and 1B, with which embodiments of the disclosure may be implemented;
FIG. 3 is a top view of the nest of FIG. 2;
FIG. 4 illustrates an environment in which a method of reading a plurality of RFID-tagged nests, such as the nest of FIGS. 2 and 3, may be performed, according to a non-limiting embodiment described herein;
FIG. 5 is a diagram of non-limiting embodiments or aspects of components of one or more devices and/or one or more systems of FIG. 4; and
FIG. 6 is a flowchart of a process for aggregating data of tagged medical injection devices and a nest, including localizing the tagged medical injection devices in the nest, according to a non-limiting embodiment described herein;
FIG. 7 illustrates a shift register for aggregating data of medical injection devices and a nest within which the devices are stored, at a first point in the method of FIG. 6;
FIG. 8 illustrates a shift register for aggregating data of medical injection devices and a nest within which the devices are stored, at a second point in the method of FIG. 6; and
FIG. 9 illustrates a shift register for aggregating data of medical injection devices and a nest within which the devices are stored, at a third point in the method of FIG. 6.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

As used herein, the terms "communication" and "communicate" may refer to the reception, receipt, transmission, transfer, provision, and/or the like of information (e.g., data, signals, messages, instructions, commands, and/or the like). For one unit (e.g., a device, a system, a component of a device or system, combinations thereof, and/or the like) to be in communication with another unit means that the one unit is able to directly or indirectly receive information from and/or transmit information to the other unit. This may refer to a direct or indirect connection that is wired and/or wireless in nature. Additionally, two units may be in communication with each other even though the information transmitted may be modified, processed, relayed, and/or routed between the first and second unit. For example, a first unit may be in communication with a second unit even though the first unit passively receives information and does not actively transmit information to the second unit. As another example, a first unit may be in communication with a second unit if at least one intermediary unit (e.g., a third unit located between the first unit and the second unit) processes information received from the first unit and communicates the processed information to the second unit. In some non-limiting embodiments or aspects, a message may refer to a network packet (e.g., a data packet and/or the like) that includes data. It will be appreciated that numerous other arrangements are possible.

As used herein, the term "computing device" may refer to one or more electronic devices that are configured to directly or indirectly communicate with or over one or more networks. A computing device may be a mobile or portable computing device, a desktop computer, a server, and/or the like that is specifically configured to provide one or more of the features and/or functions described herein. Furthermore, the term "computer" may refer to a specifically configured computing device that includes the necessary components to receive, process, and output data according to aspects described herein, and normally includes a display, a processor, a memory, an input device, and a network interface. A "computing system" may include one or more computing devices or computers. An "application" or "application program interface" (API) refers to computer code or other data sorted on a computer-readable medium that may be executed by a processor to facilitate the interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, touchscreen, etc.). Further, multiple computers, e.g., servers, or other computerized devices directly or indirectly communicating in the network environment may constitute a "system" or a "computing system".

As used herein, the term "communication network" may refer to one or more wired and/or wireless networks. For example, a communication network may include a cellular network (e.g., a long-term evolution (LTE) network, a third generation (3G) network, a fourth generation (4G) network, a fifth generation (5G) network, a code division multiple access (CDMA) network, etc.), a public land mobile network (PLMN), a local area network (LAN), a wide area network (WAN), a metropolitan area network (MAN), a telephone network (e.g., the public switched telephone network (PSTN)), a private network, an ad hoc network, an intranet, the Internet, a fiber optic-based network, a cloud computing network, and/or the like, and/or a combination of these or other types of networks.

It will be apparent that systems and/or methods, described herein, can be implemented in different forms of hardware, firmware, or a combination of hardware and software. The actual specialized control hardware or software code used to implement these systems and/or methods is not limiting of the implementations. Thus, the operation and behavior of the systems and/or methods are described herein without reference to specific software code, it being understood that software and hardware can be designed to implement the systems and/or methods based on the description herein.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least partially on" unless explicitly stated otherwise.

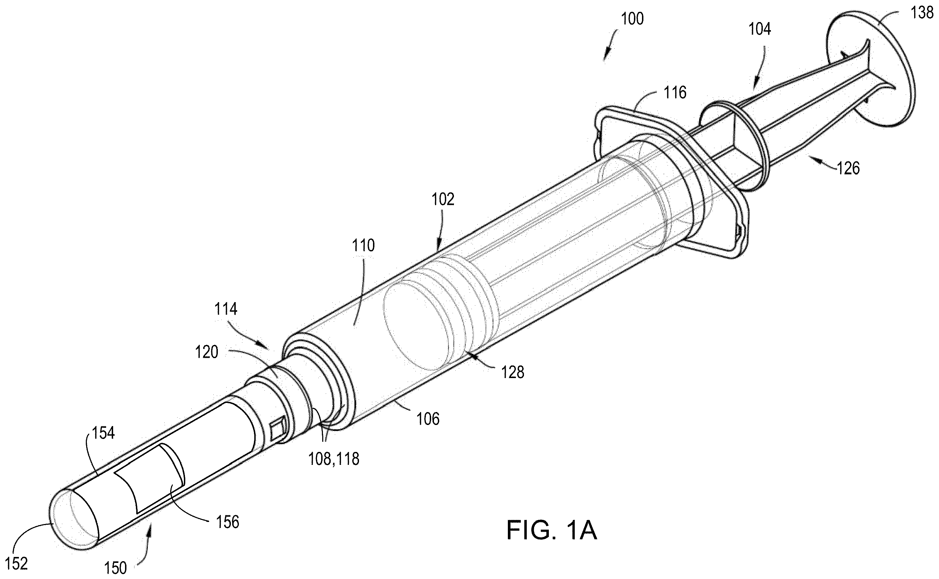

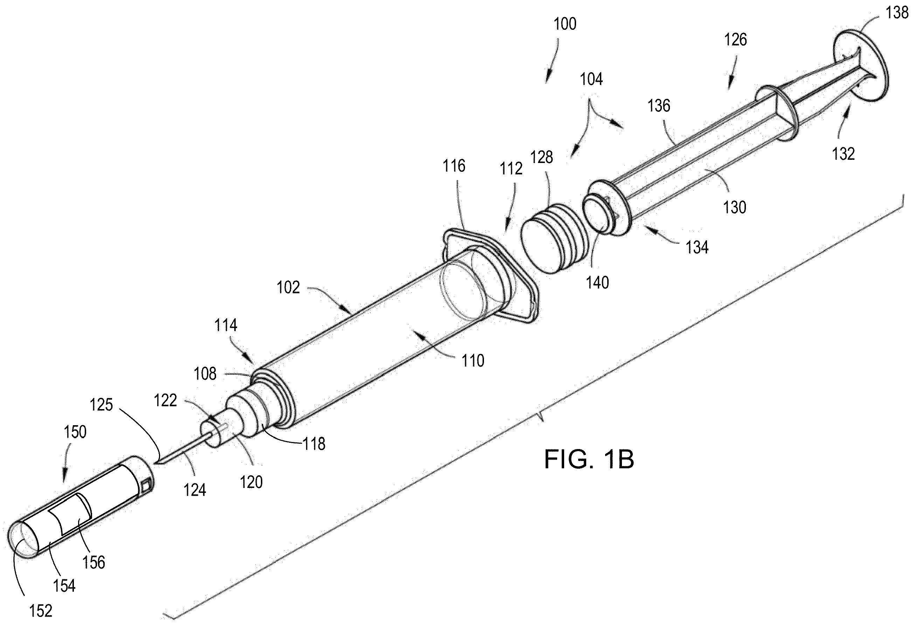

Referring to FIGS. 1A and 1B, shown is a non-limiting embodiment of a RFID-tagged medical injection device 10 with which aspects or embodiments of the disclosure may be implemented. While the medical device is shown and described hereafter as a syringe ("syringe 100"), it is recognized that other RFID-tagged medical devices, including other medical injection devices (e.g., auto-injectors) may be utilized with the system and method of the disclosure described in detail here below.

As shown in FIGS. 1A and 1B, the syringe 100 generally includes a syringe barrel assembly 102 (hereafter "syringe barrel 102") and a plunger assembly 104. The plunger assembly 104 is movable within the syringe barrel 102 along a longitudinal axis to an advanced position to facilitate administering of an injectable fluid (e.g., medication) to a patient, for example. The syringe barrel 102 is formed of a generally cylindrical outer wall 106 and an end member 108 that collectively define a chamber 110 for retaining fluid therein. The syringe barrel 102 includes an open proximal end 112 configured to receive the plunger assembly 104 therein and a distal end 114 at which end member 108 is positioned. The proximal end 112 of the syringe barrel 102 may include a flange 116 to facilitate handling and positioning of the syringe 100 and to maintain the relative position of the syringe barrel 102 with respect to the plunger assembly 104 during medication administration. At the distal end 114, the end member 108 may include a shoulder 118 which narrows with respect to the cylindrical outer wall 106, as well as a hub portion 120 extending out distally from shoulder 118. The hub portion 120 is formed as a partially hollow member that defines a channel 122 therethrough in fluid communication with the chamber 110. A needle 124 is attached to the hub portion 120 within channel 122, such as by being glued or otherwise secured to the hub portion 120, with the needle 124 extending distally from the hub 120 out to a distal needle tip 125.

The plunger assembly 104 of syringe 100 is formed of an elongate plunger rod 126 (more generally "plunger 126," as used hereafter) and a plunger head or stopper 128. The plunger 126 may include a main body 130 extending between a plunger proximal end 132 and a plunger distal end 134. In some embodiments, the main body 130 may include a plurality of elongate vanes or walls 136 extending axially along a length thereof between the plunger proximal end 132 and the plunger distal end 134. A thumb press 138 is positioned at the plunger proximal end 132 that may be engaged by a thumb (or other finger) of the user to apply a distally directed force to the plunger assembly 104 to move the plunger 126 with respect to the syringe barrel 102. In some embodiments, a flanged extension member 140 (e.g., disc-shaped flange) is positioned at the plunger distal end 134 that is configured to mate with the stopper 128. In other embodiments, the plunger distal end 134 may include a female receptacle formed therein that is configured to receive and connect to a protrusion (e.g., pin) extending out proximally from the stopper, with the protrusion and receptacle engaging via threading, for example.

As shown in FIGS. 1A and 1B, a needle shield 150 of syringe barrel 102 (i.e., of the syringe barrel assembly may be coupled to the hub portion 120 and/or shoulder 118 to protect the needle 124. According to aspects of the disclosure, the needle shield 150 may be composed of a rigid outer casing 152 that provides structure to the needle shield 150 and a compliant inner casing or "jupe" 154 that surrounds the needle 124 (when needle shield 150 is coupled to syringe barrel 102). In various embodiments, the outer casing 152 may be made of a rigid material such as polypropylene (PP), polyethylene (PE), polyethylene terephthalate (PET), polystyrene (PS) or polycarbonate (PC), while the inner casing 154 is made of a deformable material such as rubber (e.g., butyl rubber, isoprene rubber, latex rubber, silicone rubber) or a thermoplastic elastomer (TPE), as non-limiting examples.

According to aspects and embodiments of the disclosure, the needle shield 150 may include an identifying element or "tag" 156 integrated therein or applied thereto that allows for identification and/or tracking of the syringe 100. That is, the tag 156 may include or store information thereon regarding the contents of the syringe 100 (i.e., the medication or drug included therein, if a prefilled syringe) and the manufacturing history of the syringe. Accordingly, the tag 156 is considered to generally include thereon a unique device identifier (UDI) that is specific to the syringe 100. According to some aspects or embodiments of the disclosure, the tag 156 may be integrated into the needle shield 150, such as by being inlaid or otherwise disposed between the outer casing 152 and the inner casing 154 of the needle shield 150. According to other embodiments, the tag 156 may be applied to an outer surface of the needle shield 150. In each of these embodiments, the tag 156 may conform to the surface(s) or wall(s) to or within which it is disposed, such that when the tag 156 is integrated with/on a cylindrical component such as the needle shield 150, the tag 156 will have a curvature that matches that of the needle shield 150.

According to aspects of the disclosure, the tag 156 may comprise any of a number of tags that are capable of being read and that contain a UDI thereon that is specific to the syringe 100. In some embodiments, the tag 156 may comprise a RFID tag that is configured to be read by a RFID reading system to obtain the UDI for the syringe 100. In other embodiments, the tag 156 may comprise a two-dimensional code tag, such as a data matrix code tag or a QR code tag, that is configured to be read by an imaging system (i.e., camera and image processor) to obtain the UDI for the syringe 100.

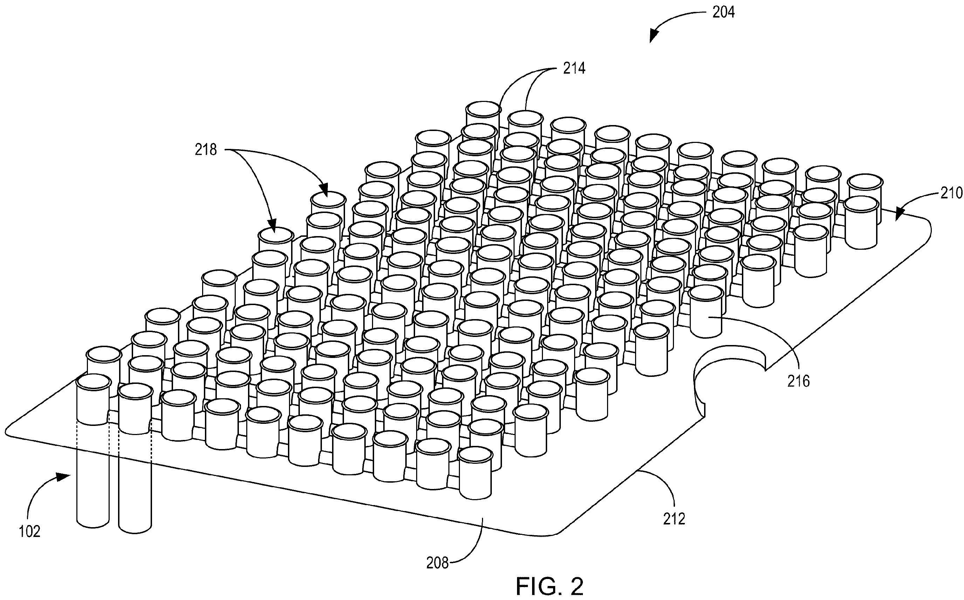

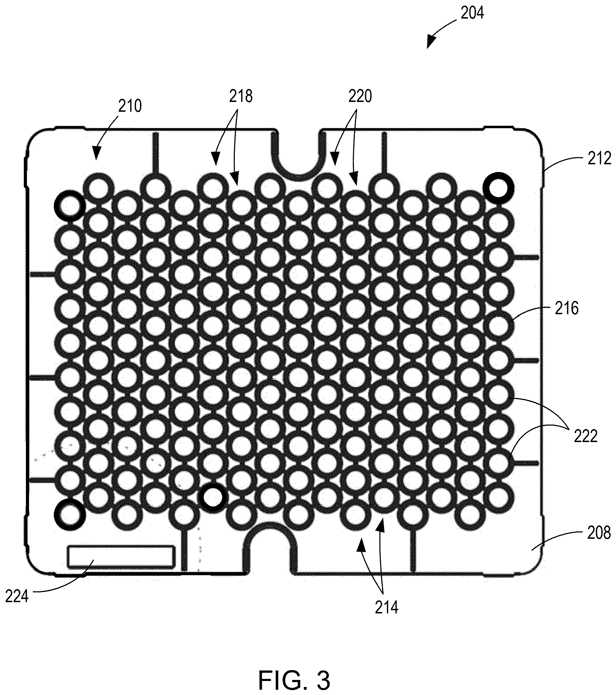

Referring to FIGS. 2 and 3, shown is a non-limiting embodiment of a nest that may be used for the storing and processing (e.g., a fill and finish process) of a plurality of medical components therein, such as syringes 100 or syringe barrel assemblies 102 thereof shown in FIGS. 1A and 1B. As shown in FIGS. 2 and 3, an exemplary nest 204 comprises a support plate 208 having a first or upper surface 210, a second or lower surface (now shown), and a peripheral edge 212 extending about the support plate 208. In some examples, the perimeter of support plate 208 is substantially rectangular in shape, while in other embodiments, the support plate 208 may be square. The nest 204 furthers comprise tubular walls, members, or ridges - referred to hereafter as "chimneys 214" - that protrude outward and upward from a plane defined by the upper surface 210 of the support plate 208. Each chimney 214 can include a cylindrical wall 216 that defines a receptacle 218 configured to receive a syringe barrel 102 therein. The receptacle 218 includes a top opening at a top end of the chimney 214 and a bottom opening at a bottom end of the chimney 214, with the receptacle sized to receive the cylindrical outer wall 106 of barrel 102 therein and the flange 116 of barrel 102 resting against an upper edge of the chimney 214. According to some aspects of the disclosure, the nest 204 can be configured to include one-hundred sixty (160) chimneys 214 for receiving syringe barrels 102, although it is recognized that another number of chimneys 214 may be provided on nest 204 (e.g., one-hundred (100) chimneys) that is dependent on the size of the syringe barrels 102 retained therein (e.g., 1-3 mL syringe barrels, 5 mL syringe barrels, 10 mL syringe barrels, etc.). The chimneys 214 may be considered as being arranged in a plurality of columns 220 (e.g., 16 columns, in the embodiment of FIGS. 2 and 3), with each of the columns 220 including a plurality of "seats" 222 that correspond to the individual chimneys 214 (e.g., 10 seats in each column, with 160 seats total, in the embodiment of FIGS. 3A and 3B).

According to aspects of the disclosure, nest 204 includes a nest tag 224 thereon, such as on the upper surface 210 of support plate 208, adjacent a corner thereof. The nest tag 224 allows for identification of the nest 204. That is, the nest tag 224 may include or store thereon a unique device identifier (UDI) that is specific to the nest 204, with it recognized that a specific nest 204 will have associated therewith the particular tagged syringe barrels 102 that are retained in the nest 204 and the location of each syringe barrel within the nest 204, so as to enable localizing of the syringe barrels 102 within nest 204, as explained in further detail below. In some embodiments, the UDI on the nest tag of nest 204 and UDIs of the tagged syringe barrels 102 retained thereby may be used for registration purposes and dispensing of a medication or drug into the syringe barrels 102 (if a prefilled syringe) during a fill and finish process.

It is recognized that, when transferring syringe barrels 102 into a nest 204 for storage/transporting thereof and later filling of the syringe barrels 102, it is desirable to be able to register the location of each specific syringe barrel 102 within the nest 204. That is, it is desirable to be able to register each syringe barrel 102 with a specific chimney 214 in the nest - i.e., the column 220 and individual seat 222 of the chimney 214. For example, a tagged syringe barrel 102 identified may be registered to "chimney #160", such that the specific location of the tagged syringe barrel 102 within nest 204 is known. According to some embodiments of the disclosure, registering of the tagged syringe barrels 102 in a nest 204 may facilitate a later filling and finishing of the syringe barrels 102 by a fill and finish machine, with the machine being controlled to fill each of the plurality of tagged medical injection devices retained in the nest with a fluid/medicament based on the registered location of each of the tagged medical injection devices within the nest and based on the UDI of each of the tagged medical injection devices retained in the nest. For example, with the UDI and the location of each of the tagged syringe barrels 102 within the nest 204 being registered a fill and finish machine may be operated to dispense a controlled amount of medicinal fluid into each of the tagged syringe barrels 102, as appropriate for each syringe barrel 102 and as determined by the information contained on the UDI thereof.

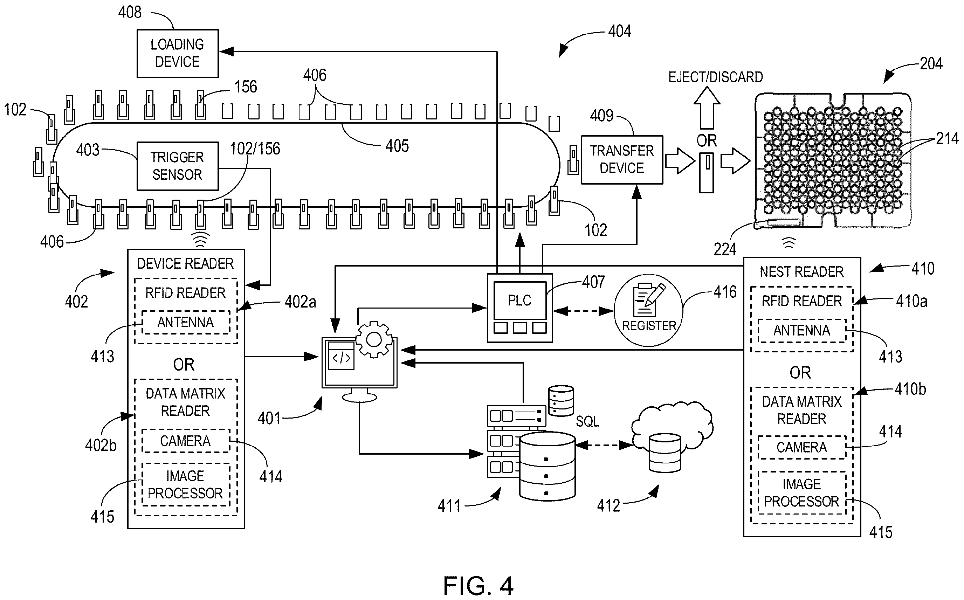

Referring now to FIG. 4, a manufacturing and reading environment or system 400 is illustrated within which or by which tagged medical injection device (such as tagged syringe barrels) may be read, in accordance with an aspect of the disclosure. As shown in FIG. 4, a plurality of tagged syringe barrels 102 may be provided to system 400, which may be configured to transfer tagged syringe barrels 102 to a nest 204 for storage/transport and subsequent filling (via an automated fill and finish machine). According to aspects of the disclosure, reading of the tagged syringe barrels 102 may be implemented as part of a process for localizing/registering tagged syringe barrels 102 as they are placed within the nest 204, including identifying the specific location (i.e., chimney 214) of each tagged syringe barrel 102 within the nest 204, when provided to system 400. As indicated above, "syringe barrels 102" may be understood to refer to an assembly of the cylindrical outer wall 106, end member 108, shoulder 118, hub portion 120, needle 124, and needle shield 150 (including RFID tag 156).

As shown in FIG. 4 system 400 may include a controller system 401, a device reader 402, a trigger sensor 403, a conveyor system 404 (including a conveyor 405, device holders 406, and programmable logic controller (PLC) 407), a loading device 408, a transfer device 409, and a nest reader 410, along with a local database system 411, and/or an external database system 412.

Controller system 401 may include one or more devices capable of receiving information and/or data from device reader 402, trigger sensor 403, conveyor system 404, loading device 408, transfer device 409, nest reader 410, local database system 411, and/or external database system 412 (e.g., via a communication network, via a direct wired and/or wireless connection, etc.) and/or communicating information and/or data to device reader 402, trigger sensor 403, conveyor system 404, loading device 408, transfer device 409, nest reader 410, local database system 411, and/or external database system 412 (e.g., via communication network, via a direct wired and/or wireless connection, etc.). For example, controller system 401 may include a computing device, such as a server, a group of servers, and/or other like devices. In some non-limiting embodiments or aspects, controller system 401 may include middleware, such as Message Queuing Telemetry Transport (MQTT) protocol-based middleware, and/or the like, configured to manage communication with and/or control of operations of device reader 402, trigger sensor 403, conveyor system 404 (i.e., PLC 407 of conveyor system 404, etc.), loading device 408, transfer device 409, nest reader 410, local database system 411, and/or external database system 412.

Device reader 402 may include one or more devices capable of receiving information and/or data from controller system 401, trigger sensor 403, conveyor system 404, loading device 408, transfer device 409, nest reader 410, local database system 411 and/or external database system 412 (e.g., via a communication network, via a direct wired and/or wireless connection, etc.) and/or communicating information and/or data to controller system 401, trigger sensor 403, conveyor system 404, loading device 408, transfer device 409, nest reader 410, local database system 411 and/or external database system 412 (e.g., via communication network, via a direct wired and/or wireless connection, etc.).

In embodiments where syringe barrels 102 include an RFID tag integrated therein, device reader 402 may comprise a RFID reader 402a configured as a passive RFID reader, an active RFID reader, or any combination thereof. The RFID reader 402a may be configured to read - from the plurality of RFID tags 224 on the syringe barrels 102 in the production line - a UDI associated with each syringe barrel 102. For example, the RFID reader 402a may be configured to attempt to read an RFID tag on a syringe barrel 102 in response to receiving a trigger signal from trigger sensor 403.

According to embodiments a RFID coupling element 413 (which may be provided as a near field antenna or other near field coupling element, but is referred to hereafter generally as "antenna 413") of RFID reader 402a may be located proximate to conveyor 405 such that, as conveyor 405 moves the syringe barrels 102 along the production line, the syringe barrels 102 are moved one-by-one to be adjacent to (and/or to be paused adjacent to for a predetermined period of time) antenna 413 of RFID reader 402a at a "reading location", and then moved past the antenna 413 of RFID reader 402. While only one antenna 413 is shown in FIG. 4, it is recognized that system 400 could include one or more additional antennas, such that a plurality of antennas 413 are associated with the RFID reader 402a.

In embodiments wherein syringe barrels 102 include a two-dimensional code tag (e.g., data matrix tag or QR code tag) integrated therein, device reader 402 may comprise an imaging system 402b configured to read - from the plurality of RFID tags 224 on the syringe barrels 102 in the production line - a UDI associated with each syringe barrel 102. For example, the imaging system 402b may be configured to attempt to read a two-dimensional code tag on a syringe barrel 102 in response to receiving a trigger signal from trigger sensor 403.

According to embodiments, imaging system 402b may comprise a camera 414 configured to acquire an image of the two-dimensional code tag of a respective medical injection device, with the camera 414 located proximate to conveyor 405 such that, as conveyor 405 moves the syringe barrels 102 along the production line, the syringe barrels 102 are moved one-by-one to be adjacent to (and/or to be paused adjacent to for a predetermined period of time) camera 414 of imaging system 402b at a "reading location", and then moved past the camera 414. Images from the camera 414 may be provided to an image processor 415 of imaging system 402b, with the image processor 415 configured to receive each image and determine the UDI of a syringe barrel 412 from the image.

Trigger sensor 403 may include one or more devices capable of receiving information and/or data from controller system 401, device reader 402, conveyor system 404, loading device 408, transfer device 409, nest reader 410, local database system 411 and/or external database system 412 (e.g., via a communication network, via a direct wired and/or wireless connection, etc.) and/or communicating information and/or data to controller system 401, device reader 402, conveyor system 404, loading device 408, transfer device 409, nest reader 410, local database system 411 (e.g., via communication network, via a direct wired and/or wireless connection, etc.).

Trigger sensor 403 may include at least one sensor (e.g., an optical sensor, a laser sensor, etc.) configured to detect or be trigged when an individual syringe barrel 102 is in a predetermined location relative to device reader 402 (e.g., when a syringe barrel 102 is at the reading location, adjacent the antenna 413 or camera 414). In response to being triggered or detecting a syringe barrel 102 in the predetermined location relative to the device reader 402, trigger sensor 403 may be configured to communicate or transmit a trigger signal to device reader 402 to trigger or cause the device reader 402 to attempt to read a tag on the syringe barrel 102.

Conveyor system 404 may include one or more devices capable of receiving information and/or data from controller system 401, device reader 402, trigger sensor 403, loading device 408, transfer device 409, nest reader 410, local database system 411 and/or external database system 412 (e.g., via a communication network, via a direct wired and/or wireless connection, etc.) and/or communicating information and/or data to controller system 401, device reader 402, trigger sensor 403, loading device 408, transfer device 409, nest reader 410, local database system 411 and/or external database system 412 (e.g., via communication network, via a direct wired and/or wireless connection, etc.).

Conveyor system 404 may include a conveyor 405 (e.g., a motorized belt conveyor, a motorized roller conveyor, a motorized chain conveyor, a motorized trolley conveyor, etc.) configured to conveys syringe barrels 102 along a path, such as a looped path. A plurality of device holders 406 are attached to the conveyor 405, with each device holder 406 configured to receive and retain a respective syringe barrel 102 therein. Conveyor 405 and holders 406 may be configured to individually move syringe barrels 102 in the production line adjacent to and past device reader 402. According to embodiments, each holder 406 may have a holder number associated therewith, with the holders thus numbered Holder_1, Holder_2,... Holder_n, and with such holder numbers being defined/stored within controller system 401, local database system 411 and/or external database system 412.

The PLC 407 of conveyor system 404 is configured to control one or more operations of conveyor system 404, including conveyor 405. The PLC 407 may be configured to track a sequential order of the holders 406 on conveyor 405, with such tracking performed via a shift register (e.g., shift register 416). The PLC 407 may be configured to receive inputs from other devices, including controller system 401, in order to aggregate data in the shift register 416, and thereby providing for registration of syringe barrels 102 with the nest 204, including the UDIs of the syringe barrels and the nest UDI, also with a location of each syringe barrel 102 within the nest 204, as explained in further detail below.

Loading device 408 may include one or more devices capable of receiving information and/or data from controller system 401, device reader 402, trigger sensor 403, conveyor system 404, transfer device 409, nest reader 410, local database system 411 and/or external database system 412 (e.g., via a communication network, via a direct wired and/or wireless connection, etc.) and/or communicating information and/or data to controller system 401, device reader 402, trigger sensor 403, conveyor system 404, transfer device 409, nest reader 410, local database system 411 (e.g., via communication network, via a direct wired and/or wireless connection, etc.).

Loading device 408 may be configured to load syringe barrels 102 into holders 406 at a "loading location" on conveyor 405 that is upstream from the device reader 402. The loading device 408 may be provided as a robotic arm or similar device that is located adjacent conveyor 405, with the loading device 408 configured to place syringe barrels 102 into empty holders 406 as they pass thereby. In embodiments where the conveying path of the conveyor is a loop-shaped path, the loading device 408 may load syringe barrels 102 into holders 406 multiple times during the course of operation of the system 400 - with a new/additional syringe barrel 102 loaded into a holder 406 after a previously loaded syringe barrel 102 has been removed from a holder 406 and transferred to nest 402.

Transfer device 409 may include one or more devices capable of receiving information and/or data from controller system 401, device reader 402, trigger sensor 403, conveyor system 404, loading device 408, nest reader 410, local database system 411 and/or external database system 412 (e.g., via a communication network, via a direct wired and/or wireless connection, etc.) and/or communicating information and/or data to controller system 401, device reader 402, trigger sensor 403, conveyor system 404, loading device 408, nest reader 410, local database system 411 (e.g., via communication network, via a direct wired and/or wireless connection, etc.).

Transfer device 409 may be configured to remove syringe barrels 102 from holders 406 at a "transfer location" on conveyor 405 that is downstream from the device reader 402. According to aspects of the disclosure, after removing a syringe barrel 102 from a respective holder 406 at the transfer location, the transfer device 409 may be selectively controlled to either transfer the syringe barrel 102 to a designated location (i.e., chimney 214) within nest 204 or to discard the syringe barrel 102 from the manufacturing system 400, as explained in further detail below. The transfer device 409 may be provided as a robotic arm or similar device that is located adjacent conveyor 405, with the transfer device 409 configured to remove a syringe barrel 102 from a holder 406 as it passes thereby and transfer the syringe barrel 102 to the nest 204 or to a discard location.

Nest reader 410 may include one or more devices capable of receiving information and/or data from controller system 401, device reader 402, trigger sensor 403, conveyor system 404, loading device 408, transfer device 409, local database system 411 and/or external database system 412 (e.g., via a communication network, via a direct wired and/or wireless connection, etc.) and/or communicating information and/or data to controller system 401, device reader 402, trigger sensor 403, conveyor system 404, loading device 408, transfer device 409, local database system 411 (e.g., via communication network, via a direct wired and/or wireless connection, etc.).

In embodiments where nest 204 includes a nest tag 224 thereon in the form of an RFID tag, nest reader 410 may comprise a RFID reader 410a configured as a passive RFID reader, an active RFID reader, or any combination thereof. The RFID reader 410a may be configured to read the RFID nest tag 224 in order to retrieve the UDI (nest UDI) associated with the nest 204. According to embodiments, a RFID coupling element 413 (which may be provided as a near field antenna or other near field coupling element, but is referred to hereafter generally as "antenna 413") of RFID reader 410a may be located proximate nest 204 to provide for reading of the nest tag 224.

In embodiments where nest 204 includes a nest tag 224 thereon in the form of a two-dimensional code (e.g., data matrix code or QR code), nest reader 410 may comprise an imaging system 410b configured to read the two-dimensional code nest tag 224 in order to retrieve the UDI associated with the nest 204. According to embodiments, imaging system 410b may comprise a camera 414 configured to acquire an image of the two-dimensional code tag 224 of nest 204, with the camera 414 located proximate to nest 204 to provide for reading of the nest tag 224.

Local database system 411 may include one or more devices capable of receiving information and/or data from controller system 401, device reader 402, trigger sensor 403, conveyor system 404, loading device 408, transfer device 409, nest reader 410, and/or external database system 412 (e.g., via a communication network, via a direct wired and/or wireless connection, etc.) and/or communicating information and/or data to controller system 401, device reader 402, trigger sensor 403, conveyor system 404, loading device 408, transfer device 409, nest reader 410,and/or external database system 412 (e.g., via communication network, via a direct wired and/or wireless connection, etc.). In some non-limiting embodiments or aspects, local database system 411 includes one or more local databases (e.g., local to and/or implemented by controller system 401, etc.). Local database system 411 may be configured to store data on the tagged nest 204 (i.e., the UDI of the nest tag 224), along with data on the tagged syringe barrels 102 that are to be transferred to and retained in nest 204 and the ordering/locations of the tagged syringe barrels 102 retained in nest 204, as explained in further detail below. In some embodiments, local database system 411 may retrieve data on tagged nest 204 and data on the tagged syringe barrels 102 retained in nest 204 from external database system 412, for subsequent storage on local database system 411.

External database system 412 may include one or more devices capable of receiving information and/or data from controller system 401, device reader 402, trigger sensor 403, conveyor system 404, loading device 408, transfer device 409, nest reader 410, and/or local database system 411 (e.g., via a communication network, via a direct wired and/or wireless connection, etc.) and/or communicating information and/or data to controller system 401,device reader 402, trigger sensor 403, conveyor system 404, loading device 408, transfer device 409, nest reader 410, and/or local database system 407 (e.g., via communication network, via a direct wired and/or wireless connection, etc.). In some non-limiting embodiments or aspects, external database system 412 includes one or more external databases (e.g., external to and/or not implemented by controller system 102, etc.). External database system 412 may be configured to store data on tagged nest 204 (i.e., the UDI of nest tag 224), along with data on the tagged syringe barrels 102 that are to be transferred to and retained in nest 204 and the ordering/locations of the tagged syringe barrels 102 retained in nest 204, as explained in further detail below.

The number and arrangement of devices shown in FIG. 4 is provided as an example. There can be additional devices, fewer devices, different devices, or differently arranged devices than those shown in FIG. 4. Furthermore, two or more devices shown in FIG. 4 can be implemented within a single device, or a single device shown in FIG. 4 can be implemented as multiple, distributed devices. Additionally, or alternatively, a set of devices (e.g., one or more devices, etc.) of system 400 can perform one or more functions described as being performed by another set of devices of system 400.

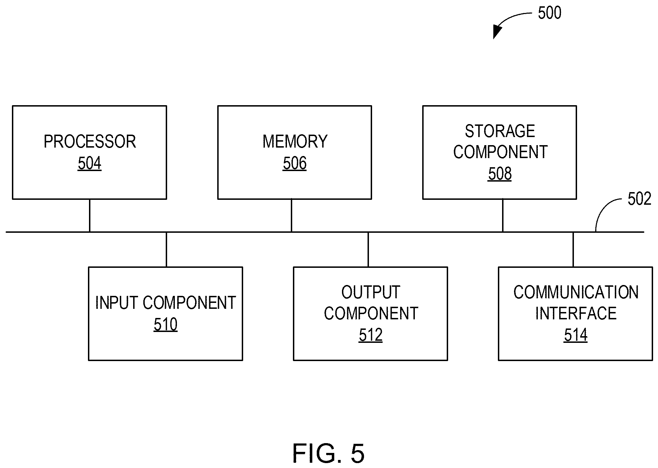

Referring now to FIG. 5, FIG. 5 is a diagram of example components of a device 500. Device 500 may correspond to one or more devices of controller system 401, device reader 402, trigger sensor 403, conveyor system 404, loading device 408, transfer device 409, nest reader 410, local database system 411 and/or external database system 412. In some non-limiting embodiments or aspects, one or more devices of controller system 401, device reader 402, trigger sensor 403, conveyor system 404, loading device 408, transfer device 409, nest reader 410, local database system 411, and/or external database system 412, may include at least one device 500 and/or at least one component of device 500. As shown in FIG. 5, device 500 may include bus 502, processor 504, memory 506, storage component 508, input component 510, output component 512, and communication interface 514.

Bus 502 may include a component that permits communication among the components of device 500. In some non-limiting embodiments or aspects, processor 504 may be specifically configured to perform one or more of the mixing aspects described in hardware, software, or a combination of hardware and software. For example, processor 504 may include a processor (e.g., a central processing unit (CPU), a graphics processing unit (GPU), an accelerated processing unit (APU), etc.), a microprocessor, a digital signal processor (DSP), and/or similar processing component (e.g., a field-programmable gate array (FPGA), an application-specific integrated circuit (ASIC), etc.) that can be programmed to perform a function. Memory 506 may include random access memory (RAM), read-only memory (ROM), and/or another type of dynamic or static storage device (e.g., flash memory, magnetic memory, optical memory, etc.) that stores information and/or instructions for use by processor 504.

Storage component 508 may store information and/or software related to the operation and use of device 500. For example, storage component 508 may include a hard disk (e.g., a magnetic disk, an optical disk, a magneto-optic disk, a solid state disk, etc.), a compact disc (CD), a digital versatile disc (DVD), a floppy disk, a cartridge, a magnetic tape, and/or another type of computer-readable medium, along with a corresponding drive.

Input component 510 may include a component that permits device 500 to receive information, such as via user input (e.g., a touch screen display, a keyboard, a keypad, a mouse, a button, a switch, a microphone, etc.). Additionally or alternatively, input component 510 may include a sensor for sensing information (e.g., a global positioning system (GPS) component, an accelerometer, a gyroscope, an actuator, etc.). Output component 512 may include a component that provides output information from device 500 (e.g., a display, a speaker, one or more light-emitting diodes (LEDs), etc.).

Communication interface 514 may include a transceiver-like component (e.g., a transceiver, a separate receiver and transmitter, etc.) that enables device 500 to communicate with other devices, such as via a wired connection, a wireless connection, or a combination of wired and wireless connections. Communication interface 514 may permit device 500 to receive information from another device and/or provide information to another device. For example, communication interface 514 may include an Ethernet interface, an optical interface, a coaxial interface, an infrared interface, a radio frequency (RF) interface, a universal serial bus (USB) interface, a Wi-Fi^{®} interface, a cellular network interface, and/or the like.

Device 500 may perform one or more processes described herein. Device 500 may perform these processes based on processor 504 (e.g., a central processing unit (CPU), a graphics processing unit (GPU), etc.) executing software instructions stored by a computer-readable medium, such as memory 506 and/or storage component 508. A computer-readable medium (e.g., a non-transitory computer-readable medium) is defined herein as a non-transitory memory device. A non-transitory memory device includes memory space located inside of a single physical storage device or memory space spread across multiple physical storage devices.

Software instructions may be read into memory 506 and/or storage component 508 from another computer-readable medium or from another device via communication interface 514. When executed, software instructions stored in memory 506 and/or storage component 508 may cause processor 504 to perform one or more processes described herein. Additionally or alternatively, hardwired circuitry may be used in place of or in combination with software instructions to perform one or more processes described herein.

Memory 506 and/or storage component 508 may include data storage or one or more data structures (e.g., a database, etc.). Device 500 may be capable of receiving information from, storing information in, communicating information to, or searching information stored in the data storage or one or more data structures in memory 506 and/or storage component 508.

The number and arrangement of components shown in FIG. 5 are provided as an example. In some non-limiting embodiments or aspects, device 500 may include additional components, fewer components, different components, or differently arranged components than those shown in FIG. 5. Additionally or alternatively, a set of components (e.g., one or more components) of device 500 may perform one or more functions described as being performed by another set of components of device 500.

According to aspects of the disclosure, it is recognized that reading of the tagged syringe barrels 102 as they move along conveyor system 404 can be used to localize the individual tagged syringe barrels 102 upon their transfer to and placement within the tagged nest 204 (that has been read by nest reader 410). Specifically, each tagged syringe barrel 102 that is read may be associated with a holder on the conveyor system 404, and upon a respective syringe barrel 102 being transferred to a next available/open chimney 214 in nest 204, the UDI for that syringe barrel 102 is associated with that specific chimney 214, so as to register the location of that syringe barrel 102 within the nest 204.

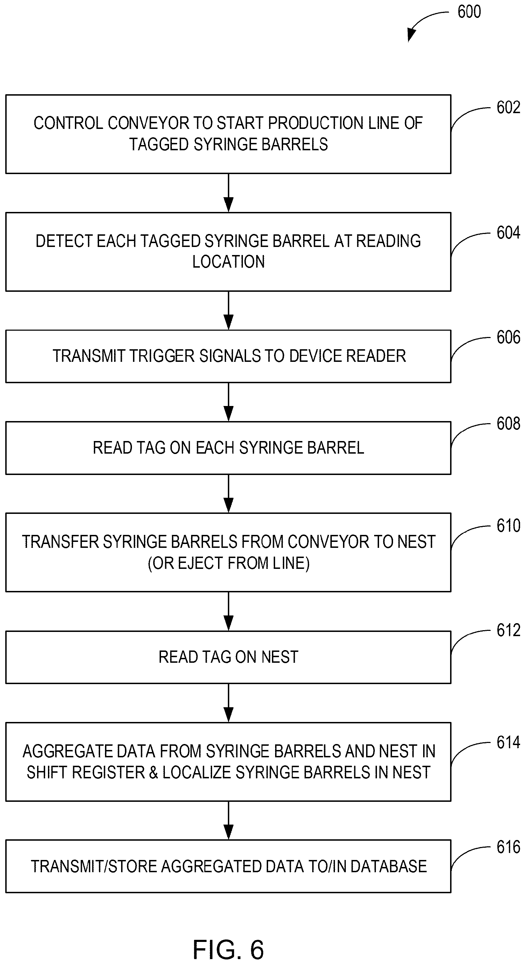

Referring now to FIG. 6, a flowchart is provided for a non-limiting embodiment or aspect of a method 600 for localizing individual tagged syringe barrels 102 within a nest 204 into which they are transferred and stored. Specifically, method 600 reads the tag 156 of each syringe barrel 102 to identify a UDI thereof and determines a placement location of each syringe barrel 102 within the nest 204 as the syringe barrels 102 are transferred from the conveyor system 404 to the nest 204. A tag 224 of the nest 204 is also read so that that UIDs of the syringe barrel and the nest are aggregated and stored in a shift register 416 and/or database (internal 411 or external 412), with the aggregated data being available during subsequent processing of the syringe barrels 102, such as a filling and finishing of the syringe barrels.

In some non-limiting embodiments or aspects, one or more of the steps of process 600 may be performed (e.g., completely, partially, etc.) by controller system 401 (e.g., one or more devices of controller system 401, etc.). In some non-limiting embodiments or aspects, one or more of the steps of process 600 may be performed (e.g., completely, partially, etc.) by another device or a group of devices separate from or including controller system 401, such as device reader 402 (e.g., one or more devices of a system of RFID reader 402, etc.), trigger sensor 403 (e.g., one or more devices of a system of trigger sensor 403, etc.), conveyor system 404 (e.g., PLC 407), loading device 408, transfer device 409, nest reader 410, local database system 411 and/or external database system 412

As shown in FIG. 6, at step 602, process 600 includes controlling a conveyor and a loading device to start a production line of tagged syringe barrels. For example, controller system 401 may control conveyor system 404 to cause conveyor 405 to start conveyor movement to move holders 406 along a conveying path and may cause loading device 408 to load tagged syringe barrels 102 into empty holders 406 as the holders pass by the loading device 408, with tagged syringe barrels 102 being conveyed along the conveyor to a reading location. As an example, controller system 401 (e.g., middleware of controller system 401, etc.) may send a control signal to conveyor system 404 (e.g., the PLC 407 of conveyor system 404, etc.) to start movement of conveyor 405 and send a control signal to device loader 408 to load syringe barrels 102 into holders 406. In such an example, controller system 401 (e.g., middleware of controller system 401, etc.) may also send a signal to arm the reader 402.

In loading syringe barrels 102 into holders 406, it is recognized that each holder 406 has a holder number (H1, H2,... Hn) associated therewith. In some embodiments, the number of holders 406 provided in conveyor system 404 may be less than the number of chimneys 214 in the nest 204. In other embodiments, the number of holders 406 provided in conveyor system 404 may be equal to or greater than the number of chimneys 214 in the nest 204.

As shown in FIG. 6, at step 604, process 600 includes detecting a tagged syringe barrel 102 in a predetermined location relative to a device reader. For example, for each tagged syringe barrel 102 in the production line, trigger sensor 403 may detect when that tagged syringe barrel 102 is in a predetermined location relative to the device reader 402 (i.e., at the reading location). In some embodiments, step 604 will thus determine when that tagged syringe barrel 102 is in a predetermined location relative to an antenna(s) 413 of an RFID reader 402a. In other embodiments, step 604 will thus determine when that tagged syringe barrel 102 is in a predetermined location relative to a camera 414 of an imaging system 402b.

As shown in FIG. 6, at step 606, process 600 includes transmitting a trigger signal to a device reader. For example, for each tagged syringe barrel 102 of the plurality of tagged syringe barrel 102 in the production line, trigger sensor 403 may, in response to detecting that the tagged syringe barrel 102 is at a predetermined location relative to device reader 402 in the production line (i.e., at the reading location), transmit a trigger signal to device reader 402 to trigger or cause the device reader 402 to read the tag 156 of the syringe barrel 102.

As shown in FIG. 6, at step 608, process 600 includes reading a tag on a syringe barrel. For example, for each syringe barrel 102 of the plurality of syringe barrels 102 in the production line, device reader 402 may, in response to receiving the trigger signal from trigger sensor 403 (e.g., corresponding to that syringe barrel), read the tag 156 on that syringe barrel 102.

In one embodiment, where tag 156 is an RFID tag, an RFID reader 402a may transmit radio waves via antenna(s) 413 to RFID tag 156. When at the reading location, radio waves from the antenna 413 will activate the RFID tag 156. Responsive to receiving the radio waves, the device RFID tag 156 may transmit a (backscattered) signal that includes an identifier (i.e., UDI) associated with that syringe barrel 102, back to the antenna 413, to be read by RFID reader 402a.

In another embodiment, where tag 156 is a two-dimensional code tag such as a data matrix or QR code tag, an imaging system 402b may acquire an image of the two-dimensional code tag 156 via a camera 414. When at the reading location, the camera 414 acquires an image of the two-dimensional code tag 156, with the image being transmitted back to an image processor 415 of imaging system 402b for processing of the image.

The device reader 402 may read, from the tags 156 on syringe barrels 102 in the production line, a plurality of identifiers (e.g., a plurality of UDIs, etc.) associated with the plurality of syringe barrels 102 (e.g. as the plurality of syringe barrels 102 in the production line are individually moved adjacent to device reader 402 by conveyor 405, etc.). In such an example, for each syringe barrel 102 in the production line, device reader 402 may transmit or stream, to controller system 401 (e.g., to middleware of controller system 401, etc.) the information or data read from the tag 156 (i.e., the UDI).

In the event that device reader 402 is not able to read a tag 156 of a respective syringe barrel 102 upon reaching the reading location - either due to the tag 156 being defective or a syringe barrel 102 being retained incorrectly within (or missing from) the holder 406, the device reader 402 may indicate a failed status for that syringe barrel 102. In such an example, for each defective/missing syringe barrel 102 in the production line, device reader 402 may transmit or stream, to controller system 401 (e.g., to middleware of controller system 401, etc.) a "Failed" status indicator.

In addition to the controller system 401 receiving information or data read from the tags 156 (i.e., the UDI) of syringe barrels 102 from the device reader 402, the controller system 401 also transmits the tag data to the PLC 407. For each tagged syringe barrel read by device reader 402 at the reading location, the PLC 407 receives (from controller system 401) the UDI (or Failed status indicator) for that syringe barrel 102, with the UDI provided to shift register 416 - where the UDI (or Failed status indicator) of each syringe barrel 102 is associated with the holder number of the holder 406 in which that syringe barrel 102 is retained.

As shown in FIG. 6, at step 610, process 600 includes transferring syringe barrels from the conveyor to a nest. For example, for each syringe barrel 102 of the plurality of syringe barrels 102 in the production line, the syringe barrel 102 may be transferred from the conveyor 405 to nest 204 by transfer device 409. The transfer device 409 may comprise a robotic pick-and-place device, as a non-limiting example, that is positioned adjacent conveyor 405 at a transfer location that is downstream along the conveying path from the reading location. As a holder 406 holding a respective syringe barrel 102 moves adjacent the transfer device 409, the transfer device 409 operates to remove the syringe barrel 102 from the holder 406 and transfer the syringe barrel 102 to an open/available chimney 214 in the nest 204. According to embodiments, the transfer device 409 may be operated according to pre-programmed instructions (e.g., instructions stored in PLC 407, with the PLC 407 controlling the transfer device 409), to place a syringe barrel 102 in a first/next available chimney 214 in the nest 204, as the nest 204 is sequentially filled with syringe barrels 102.

In transferring each respective syringe barrel 102 from the conveyor 405 to the nest 204, an initial determination is made by PLC 407 regarding the syringe barrel 102 to be transferred has a UDI associated therewith that was previously read by device reader 402 - i.e., whether device reader 402 was able to successfully read the tag 156 for that syringe barrel 102. The PLC 407 may access the shift register 416 to determine whether a UDI or a Failed status indicator is stored therein for the syringe barrel 102 retained in the holder 406 (with known (holder number) at the transfer location.

If the information stored in the shift register for the subject syringe barrel 102 (and associated holder number) indicates that a UDI is registered with that syringe barrel, then the transfer device 409 operates to transfer the syringe barrel 102 to the first available chimney 214 in the nest 204. Upon transfer, the location of the chimney 214 (e.g., the column and seat number of the chimney 214) is associated with the UDI of the syringe barrel 102, so as to register the location of the syringe barrel 102 within the nest 204.

If the information stored in the shift register for the subject syringe barrel 102 (and associated holder number) indicates that no UDI is registered with that syringe barrel - but that instead a Failed status indicator is registered with that syringe barrel - then the transfer device 409 operates to remove the syringe barrel 102 from the production line. In one embodiment, transfer device 409 may act to eject or otherwise remove the syringe barrel 102 from the holder 406, with the syringe barrel 102 being ejected to a designated discard location.

As shown in FIG. 6, at step 612, process 600 includes reading a nest tag on the nest, in order to associate the nest with the syringe barrels being stored therein. For example, a nest tag 224 on nest 204 may be read by nest reader 410, to associate the nest 204 with the syringe barrels 102 being stored therein. According to embodiments, the nest tag 224 may be in the form of an RFID tag - such that nest reader 410 is a RFID reader 410a with antenna 413 - or a two-dimensional code (e.g., data matrix or QR code) - such that nest reader 410 is an imaging system 410b with a camera 414 and image processor 415. The nest reader 410 retrieves (from nest tag 224) the UDI associated with the nest 204, and transmits the nest UDI to controller system 401. The controller system 401, in turn, may provide the nest UDI to PLC 407 - with the PLC 407 providing the nest UDI to the shift register 416.

According to embodiments, it is recognized that the performing of step 612 could take place prior to transferring of syringe barrels 102 into nest 204 or upon completion of the transferring of syringe barrels 102 into nest 204.

As shown in FIG. 6, subsequent to reading of the nest tag and filling of the nest 204 with syringe barrels, the process 400 continues at step 614. At step 614, data from the syringe barrels and the nest within which the syringe barrels are loaded is aggregated by a controller system and/or PLC of a conveyor system, with the location of each of the plurality of tagged syringe barrels being registered within the nest. For example, upon reading of the nest tag 224 and filling of the nest 204 with syringe barrels 102 (after reading of the tag 156 of each syringe barrel 102), controller system 401 operably communicates with the PLC 407 of conveyor system 404, and PLC 407 registers the location of each of the plurality of RFID-tagged syringe barrels 102 within each the nest 204. The registered location of each of the plurality of tagged syringe barrels 102 may indicated the column and seat of the chimney 214 within which each syringe barrel 102 is retained.

In some embodiments, the process 400 continues at step 616, where the aggregated syringe barrel data and nest data, including the registering of the location of each syringe barrel in the nest, may be transmitted to and stored in a database. For example, upon the shift register 416 of PLC 407 aggregating syringe barrel data and nest data therein the aggregated data may be stored in local database system 411 and/or in external database system 412, including the UDIs of the tagged syringe barrels 102 retained within the nest 204 and a location of each of the plurality of tagged syringe barrels 102 within the nest 204.

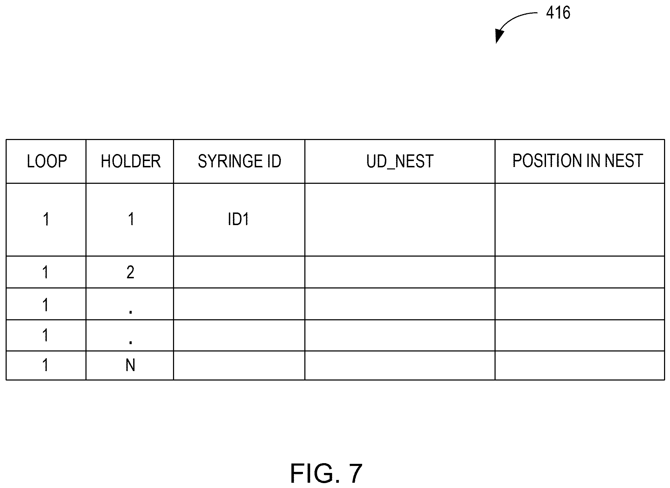

Referring now to FIGS. 7-9 illustrations of the data provided to and contained within shift register 416 during various stages of the method 600 are provided, with the data including aggregated syringe barrel UDI data and nest UDI data, along with data on various aspects/features of the system 400. As shown in the figures, the shift register may generally comprise a table-like format, with rows (X) and columns (Y) included therein into which data is entered, with each column directed to a separate parameter or data type, and with each row directed to a distinct data entry into the shift register 416. As shown in FIGS. 7-9, the parameters/data types that may be included in the shift register 416 may include:

Loop Number - when the total number of chimneys 214 in nest 204 is higher than the number of holders 406 in conveyor system 404, multiple loops of the conveyor 405/holders 406 about the conveying path must be performed, in order to provide a sufficient number of syringe barrels 102 to fill the nest. The loop number in the shift register 416 will indicate in which loop number (1, 2,... k) a syringe barrel 102 was transferred to nest 204.

Holder Number - indicates a designated number for the holder 406 in which a syringe barrel 102 that is read is retained.

Syringe ID - indicates the UDI of a syringe barrel 102 that is read.

Nest ID - indicates the UDI of the nest 204 that is read, into which the syringe barrels 102 are being loaded.

Position in Nest - indicates the location within the nest 204 that a respective syringe barrel 102 is retained, with the location comprising a column and seat.

Referring now to FIG. 7, the PLC shift register 416 is shown in a state that would be present upon a first syringe barrel 102, ID_1, being read by device reader 402, as performed at step 608 of the method 600. The tagged syringe barrel 102 is read to retrieve the UDI therefrom, with the UDI associated with a respective holder number (and loop number) in the shift register 416. While not shown in FIG. 7, it is recognized that if device reader 402 was unable to read the first syringe barrel 102, a Failed status indicator would be entered into the shift register for that syringe barrel, rather than a UDI for the syringe barrel. That Failed status indicator would then be used to flag that syringe barrel 102, such that the syringe barrel would be removed/ejected (by transfer device 409) rather than be loaded into the nest 204, as explained in detail above.

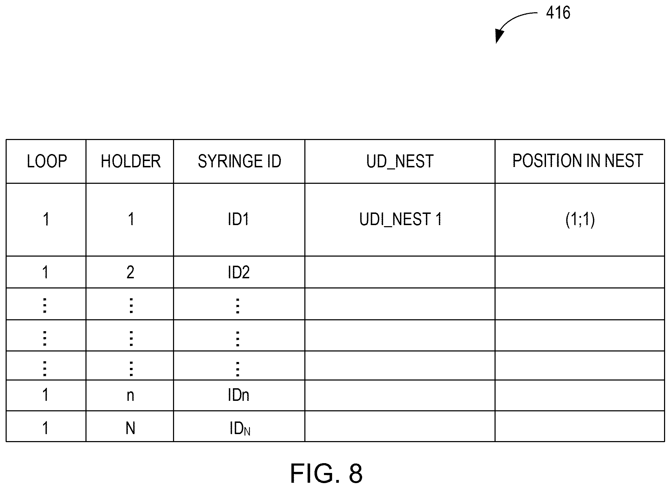

Referring next to FIG. 8, the PLC shift register 416 is shown in a state that would be present upon the first syringe barrel 102, ID_1, being transferred to the nest 204 by transfer device 409 (and with additional syringe barrels 102 continuing to be read by device reader 402 as they proceed past the reading location), and upon the nest tag 224 being read by nest reader 410, as performed at steps 610 and 612, respectively, of the method 600. The tagged nest 204 is read to retrieve the UDI therefrom, with the nest UDI associated with the syringe barrel UDI in the shift register 416. Additionally, as the first syringe barrel 102, ID_1, is placed in the nest 204 - at a known/pre-determined first available chimney 214 in the nest 204, the UDI for the first syringe barrel 102 is associated in the shift register 416 with the chimney 214 in which the syringe barrel is placed (i.e., column and seat, such as (1; 1), as shown in FIG. 8).

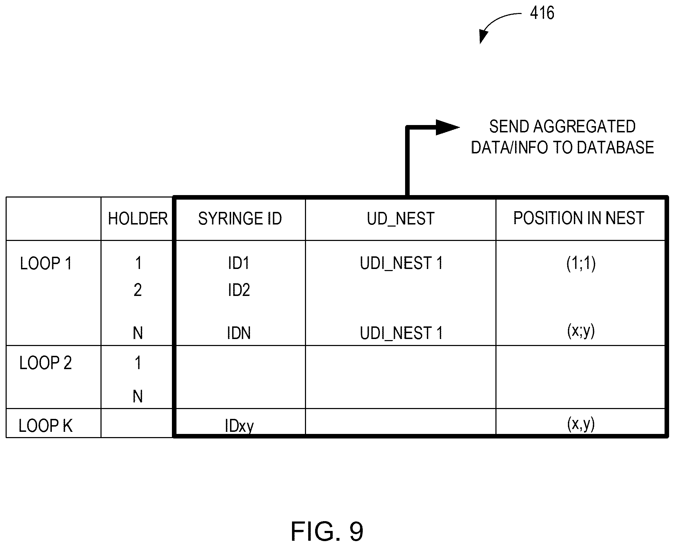

Referring next to FIG. 9, the PLC shift register 416 is shown in a state that would be present upon the nest 204 having been completely loaded/filled with syringe barrels 102 (ID_1 through ID_N). As described with respect to FIG. 8, each syringe barrel 102 has a nest UDI associated therewith, as well as a specific chimney 214 in which that syringe barrel is placed (i.e., column and seat (x; y), as shown in FIG. 9). Additionally, a holder number and loop number are also associated with all syringe barrels 102 that were loaded into nest 204. A complete set of aggregated data is thus provided, as performed at step 614, of the method 600 - with the aggregated data including a nest UDI for the nest 204, device UDIs for all syringe barrels 102 retained in nest 204, and the chimney location for each syringe barrel 102 in nest 204.

As further indicated in FIG. 9, the device and nest data aggregated in the shift register 416 may be transmitted to and stored in a database, as performed at step 616, of the method 600. According to embodiments, the aggregated data may be sent to and stored in local database system 411 and/or external database system 412, with the aggregated data including the UDIs of the tagged syringe barrels 102 retained within the nest 204 and a location of each of the plurality of tagged syringe barrels 102 within the nest 204,

According to aspects of the disclosure, the aggregated device and nest data may be used, for example, for controlling a fill and finish machine to fill each of the tagged syringe barrels 102 retained in the nest 204 with fluid, based on the registered location of each of the syringe barrels 102 within the nest 204 and based on the UDI of each of the syringe barrels 102. For example, with the UDI and the location of each of the tagged syringe barrels 102 within nest 204 being registered in/with the PLC 407 (i.e., shift register 416), a fill and finish machine may be operated to dispense a controlled amount of medicinal fluid into each of the tagged syringe barrels 102, as appropriate for each syringe barrel 102 and as determined by the information contained on the UDI thereof.

Beneficially, embodiments of the invention thus provide a system and method for localizing individual tagged syringe barrels within a nest, as the syringe barrels are loaded into the nest.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A system for localizing tagged medical injection devices in a nest, the system comprising:
a conveyor system configured to convey a plurality of tagged medical injection devices along a conveying path, the conveyor system comprising a plurality of holders, with each of the plurality of holders identified by a holder number and configured to receive a respective tagged medical injection device of the plurality of tagged medical injection devices;
a device reader positioned at a reading location along the conveying path, the device reader configured to perform a reading of a tag of each of the plurality of tagged medical injection devices upon each respective tagged medical injection device passing the reading location, wherein reading the tag comprises reading a unique device identifier (UDI) from the tag;
a transfer device positioned adjacent the conveyor system, at a location downstream from the reading location, the transfer device configured to individually transfer each of the plurality of tagged medical injection devices from the conveyor system to a nest, the nest including a nest tag having a nest UDI associated therewith; and
at least one processor coupled to a memory and configured to:
receive the UDI for a respective tagged medical injection device from the device reader; and
upon the tagged medical injection device being transferred to the nest, associating the UDI of the tagged medical injection device with a location in the nest at which the tagged medical injection device is stored and with the nest UDI, so as to register the location of each of the plurality of tagged medical injection devices within the nest.

2. The system of claim 1, wherein the at least one processor is configured to associate the UDI for a respective tagged medical injection device with the holder number of the holder carrying the tagged medical injection device.

3. The system of claim 2, wherein the at least one processor is further configured to:
receive a fail signal from the device reader if the device reader is unable to read the tag of a respective tagged medical injection device as it passes the reading location; and
associate the fail signal with the holder number of the holder that passed the reading location with no tag reading occurring.

4. The system of claim 3, wherein the at least one processor is operably connected to the transfer device, and wherein the at least one processor is further configured to:
cause the transfer device to transfer a respective tagged medical injection device from a respective holder to the nest, when the holder number for the holder is determined to have a UDI for a tagged medical injection device associated therewith; or
cause the transfer device to discard a respective tagged medical injection device from a respective holder, when the holder number for the holder is determined to have a fail signal associated therewith.

5. The system of any of claims 1-4, wherein the nest comprises a support plate and a plurality of chimneys extending outwardly from the support plate, each of the plurality of chimneys configured to retain a tagged medical injection device therein, the plurality of chimneys arranged in a plurality of columns, with each of the plurality of columns comprising a plurality of seats.

6. The system of claim 5, wherein associating the location in the nest at which the tagged medical injection device is stored with the UDI for the tagged medical injection device comprises registering the column and seat at which the tagged medical injection device is stored with the UDI.

7. The system of claim 5 or claim 6, wherein the number of chimneys in the nest is greater than the number of holders in the conveyor system, and wherein the conveying path comprises a loop, and wherein each of the holders passes by the reading location once per time traversing about the loop, with a respective tagged medical injection device being loaded into a respective holder at a beginning of the loop.

8. The system of claim 7, wherein the processor is configured to generate a shift register comprising a plurality of data entries, wherein each data entry comprises a loop number, the holder number, the medical injection device UDI, the nest UDI, and the location in the nest for the medical injection device associated with the medical injection device UDI.

9. The system of any of claims 1-8, further comprising at least one trigger sensor configured to detect when a respective tagged medical injection device is at the reading location;
wherein, for each tagged medical injection device on the conveyor system, the at least one sensor is configured to transmit a trigger signal to the device reader to cause the device reader to read the tag of the tagged medical injection device in response to detecting that the medical injection device is at the reading location; and
wherein the device reader is configured to read the tag on the respective tagged medical injection device in response to receiving the trigger signal from the at least one sensor.

10. The system of any of claims 1-9, wherein the tag comprises a RFID tag, and wherein the device reader comprises:
an antenna configured to receive a signal response from the RFID tag of a respective medical injection device; and
a RFID reader operably connected to the antenna, to receive the signal response and read the UDI therefrom.

11. The system of any of claims 1-9, wherein the tag comprises a two-dimensional code tag, and wherein the device reader comprises an imaging system including:
a camera configured to acquire an image of the two-dimensional code tag of a respective medical injection device; and
an image processor configured to receive the image and determine the UDI from the image.

12. The system of any of claims 1-11, further comprising a loading device positioned adjacent the conveyor system, at a location upstream from the reading location, the loading device configured to load a respective tagged medical injection device into a respective holder.

13. The system of any of claims 1-12, further comprising a nest reader configured to read the nest tag to obtain the nest UDI, and wherein the processor receives the nest UDI from the nest reader.

14. The system of any of claims 1-13, wherein the plurality of tagged medical injection devices comprise a plurality of syringe barrels.

15. A method for localizing tagged medical injection devices in a nest, the method comprising:
conveying each of a plurality of tagged medical injection devices, via a conveyor system including a plurality of holders, along a conveying path, each of the plurality of tagged medical injection devices comprising a tag thereon having a device unique identifier (UDI);
performing a reading of each of the plurality of tagged medical injection devices at a reading location along the conveying path, via a device reader, as each respective tagged medical injection device passes the reading location;
associating, via at least one processor, the UDI of each respective tagged medical injection device with a holder number of a respective holder retaining the tagged medical injection device;
transferring the each of the plurality of tagged medical injection devices from the conveyor system to a nest, via a transfer device positioned adjacent the conveyor system and at a location downstream from the reading location, the nest including a nest tag having a nest UDI associated therewith; and
as each of the plurality of tagged medical injection devices is being transferred to the nest, associating, via the at least one processor, the UDI of a respective tagged medical injection device with a location in the nest at which the tagged medical injection device is stored and with the nest UDI, so as to register the location of each of the plurality of tagged medical injection devices within the nest.

16. The method of claim 15, wherein the nest comprises a plurality of chimneys each configured to retain a tagged medical injection device therein, the plurality of chimneys arranged in a plurality of columns, with each of the plurality of columns comprising a plurality of seats, and wherein registering the location of each of the plurality of tagged medical injection devices comprises registering the column and seat of each of the plurality of tagged medical injection devices.

17. The method of claim 15 or claim 16, wherein performing a reading of each of the plurality of tagged medical injection devices comprises one of:
reading an RFID tag of each of the plurality of tagged medical injection devices via an RFID reader; or
reading a two-dimensional code on each of the plurality of tagged medical injection devices via an imaging system.

18. The method of any of claims 15-17, further comprising:
generating a fail signal from the device reader if the device reader is unable to read the tag of a respective tagged medical injection device as it passes the reading location; and
associating, via the at least one processor, the fail signal with the holder number of the holder that passed the reading location with no tag reading occurring.

19. The method of claim 18, further comprising:
causing, via the at least one processor, the transfer device to transfer a respective tagged medical injection device from a respective holder to the nest, when the holder number for the holder is determined to have a UDI for a tagged medical injection device associated therewith; or
causing, via the at least one processor, the transfer device to discard a respective tagged medical injection device from a respective holder, when the holder number for the holder is determined to have a fail signal associated therewith.

20. The method of any of claims 15-19, wherein in associating the UDI of a respective tagged medical injection device with the location in the nest at which the tagged medical injection device is stored and with the nest UDI, the at least one processor generates a shift register comprising a plurality of data entries, wherein each data entry comprises the holder number, the medical injection device UDI, the nest UDI, and the location in the nest for the medical injection device associated with the medical injection device UDI.

21. The method of claim 20, wherein conveying each of the plurality of tagged medical injection devices along the conveying path comprises conveying each of the plurality of tagged medical injection devices along a loop, and wherein each of the holders passes by the reading location once per time traversing about the loop, with a respective tagged medical injection device being loaded into a respective holder at a beginning of the loop; and
wherein each data entry in the indexing shift register further comprises a loop number.

22. The method of any of claims 15-21, further comprising:
detecting when a respective tagged medical injection device is at the reading location using at least one trigger sensor; and
in response to detecting that the tagged medical injection device is at the reading location, transmitting a trigger signal from the at least one trigger sensor to the device reader to cause the device reader to read the tag of a respective tagged medical injection device.
